(19) Europäisches Patentamt
European Patent Office
Office européen des brevets

(11) **EP 0 616 517 B1**

(12) **EUROPÄISCHE PATENTSCHRIFT**

(45) Veröffentlichungstag und Bekanntmachung des Hinweises auf die Patenterteilung:
**09.04.1997 Patentblatt 1997/15**

(21) Anmeldenummer: **92924633.8**

(22) Anmeldetag: **01.12.1992**

(51) Int. Cl.$^6$: **A61K 7/04**, A45D 31/00

(86) Internationale Anmeldenummer:
**PCT/EP92/02771**

(87) Internationale Veröffentlichungsnummer:
**WO 93/11734 (24.06.1993 Gazette 1993/15)**

(54) **SELBSTKLEBENDES LAMINAT FÜR FUSS- UND FINGERNÄGEL**
SELF-ADHESIVE LAMINATE FOR TOENAILS AND FINGERNAILS
STRATIFIE AUTOADHESIF POUR ONGLES DES DOIGTS ET DES ORTEILS

(84) Benannte Vertragsstaaten:
**AT BE CH DE DK ES FR GB GR IE IT LI LU MC NL PT SE**

(30) Priorität: **12.12.1991 DE 4140888**

(43) Veröffentlichungstag der Anmeldung:
**28.09.1994 Patentblatt 1994/39**

(73) Patentinhaber: **LTS LOHMANN Therapie-Systeme GmbH**
**56567 Neuwied (DE)**

(72) Erfinder:
- **HOFFMANN, Hans-Rainer**
  **D-5450 Neuwied 22 (DE)**
- **VON KLEINSORGEN, Reinhard**
  **D-56170 Bendorf (DE)**
- **SIMON, Hans, Günther**
  **D-5533 Hillesheim (DE)**
- **STEINBORN, Dorothea**
  **D-5450 Neuwied 22 (DE)**

(74) Vertreter: **Flaccus, Rolf-Dieter, Dr.**
**Patentanwalt**
**Sperlingsweg 32**
**50389 Wesseling (DE)**

(56) Entgegenhaltungen:
**WO-A-92/02204** **DE-A- 337 458**

EP 0 616 517 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.14.1/3.4

## Beschreibung

Die Erfindung betrifft ein selbstklebendes an Fuß- und/oder Fingernägel anformbares Laminat, enthaltend

a) eine filmbildende Polymerschicht mit 1-30% eines Weichmachers, bezogen auf die Polymeren,
b) eine darauf befindliche Haftkleberschicht,
c) eine die Haftkleberschicht abdeckende, ablösbare Trägerfolie;
d) eine ebenfalls ablösbare, die filmbildende Polymerschicht a) mindestens vollflächig abdeckende Schutzschicht, wobei
e) die Schutzschicht gegenüber den übrigen Bestandteilen des Laminats und den bei der Herstellung des Laminats verwendeten Materialien resistent ist und
f) die Adhäsion der Schutzschicht d) an der Polymerschicht a) durch eine abhäsive Ausrüstung auf der beschichteten Seite geringer als die Adhäsion zwischen der Haftkleberschicht b) und der Trägerfolie c) ist sowie

seine Verwendüng zur Anbringung auf Fuß- bzw. Fingernägeln als künstliche Nägel.

Es ist bekannt, Nägel mittels lösungsmittelhaltigen Polymerlacken zur Erzielung eines kosmetischen Effektes zu behandeln (Nagellack). Ebenfalls ist bekannt, einem solchen Lack einen antimykotisch wirksamen Stoff bzw. einen den Nagel pflegenden Wirkstoff zuzusetzen.
Auch ist es allgemein bekannt, wieviel Mühe es bereitet, einen solchen Nagellack mit dem gewünschten Erscheinungsbild aufzutragen. (Zeit, Trocknung des Lacks, Übung, äußeres Erscheinungsbild).

Der Gehalt der bekannten Nagellacke an organischen Lösungsmitteln stellt angesichts des hohen weltweiten Verbrauchs eine erhebliche Umweltbelastung dar, die aus Gründen des Umweltschutzes vermieden oder wenigstens verrringert werden sollte. Allein schon aus diesem Grund sollte der Einsatz einer dekorativen Folie zur Applikation auf Nägel, verbunden mit den vom Verbraucher geforderten Eigenschaften wie Glanzeffekt, Farbeffekt, Haltbarkeit, Tragbarkeit und Natürlichkeit als Ersatz für den lösungsmittelhaltigen Lack als sinnvolle Alternative anerkannt werden.
Der Einsatz künstlicher Nägel, wie auch immer mit dem Nagel verbunden, ist bekannt.
Aus der DE-OS 23 62 818 ist es auch bekannt, Nägel mit einer haftenden Folie aus PVC zu bekleben. Ein derartiges System hat jedoch im Markt keine Akzeptanz gefunden, weil PVC als Polymermaterial nicht die Verbrauchererwartungen hinsichtlich Glanz, Härte, Flexibilität und Haltbarkeit erfüllt. Die hohe Reißfestigkeit der PVC-Folie oder ähnlicher Polymerfilme wie Polyethylen, Polypropylen oder Polyester erschweren eine Anformbarkeit der Folie auf den Nagel durch einfaches Abscheren und bergen die Gefahr der Verletzung des Nagelbettes.
Die Anformbarkeit auf dem Nagel wie auch die Stabilität im Hinblick auf die Applikationsfähigkeit stehen in einen direkten Zusammenhang mit der Sprödigkeit des Laminats, wobei festgestellt werden mußte, daß die idealen Eigenschaften eines Nagellacklaminats (Laminat auf Basis Nitrocellulose - käuflicher Nagellack) Stabilitätsprobleme aufwirft, derart, daß das Laminat bei Lagerung brüchig wird (austrocknet) und nicht mehr zu applizieren ist.

Des weiteren ist aus der DE-OS 33 37 458 A1 eine Nageldekorschicht oder -folie bekannt, die zur Umgehung der mit der Verwendung von Nagellacken auftretenden Nachteile eine oder mehrere Lack-, Folien-, Vlies-, Gewebe- oder Klebstoffschichten oder eine Kombination aus diesen aufweist und die mittels eines geeigneten Haftvermittlers durch Anwendung von Druck und/oder Wärme und/oder Strahlung und/oder einer chemischen Reaktion entweder von einem Trägermaterial oder direkt auf den Nagel übertragen werden. Diese Nageldekorschicht stellt somit ein selbstklebendes an Fuß- und Fingernägel anformbares Laminat dar, das eine filmbildende Polymerschicht, eine auf dieser angeordnete Haftkleberschicht, eine die Haftkleberschicht abdeckende, wieder ablösbare Trägerfolie sowie eine ebenfalls ablösbare, die Polymerschicht mindestens vollflächig abdeckende Schutzschicht aufweist.

In der nachveröffentlichten WO 92/02204 ist ein selbstklebendes, weichmacherhaltiges Laminat beschrieben, das an Fuß- und/oder Fingernägel anformbar ist. Dieses Laminat besteht aus einer filmbildenden Polymerschicht mit 30% eines Weichmachers, einer darauf befindlichen Haftkleberschicht, einer die Haftkleberschicht abdeckenden, wieder ablösbaren Trägerfolie, einer ebenfalls ablösbaren, die filmbildende Polymerschicht mindestens vollflächig abdeckenden Schutzschicht, wobei die Schutzschicht gegenüber den übrigen Bestandteilen des Laminats und den bei der Herstellung des Laminats verwendeten Materialien resistent und die Adhäsion der Abdeckschicht (Schutzschicht) an der Polymerschicht durch eine abhäsive Ausrüstung auf der beschichteten Seite geringer als die Adhäsion zwischen der Haftkleberschicht und der Schutzschicht (Trägerfolie) ist. Die abhäsive Ausrüstung der Abdeckschicht (Schutzschicht) ist durch eine Beschichtung mit einem Silikon erreicht worden, wobei infolge Rakelauftrag in der Polymerschicht feine Rillenstrukturen den Glanzeffekt erheblich zu stören vermögen.

Aufgabe der vorliegenden Erfindung war es daher, ein selbstklebendes Laminat zu entwickeln, welches vergleichbar ist mit den im Handel befindlichen Nagellacken auf Basis von Lösungsmitteln, vom Verbraucher gewünschte Eigenschaften wie Glanz, Härte, Flexibilität und Haltbarkeit auf dem Nagel ohne Fremdkörpergefühl aufweist, und auf dem Nagel ohne jede Schwierigkeit anformbar ist, das zusammen mit den vorgenannten Eigenschaften eine ausreichende Stabilität gegen Austrocknung und die dadurch verursachte hohe Brüchigkeit besitzt und bei dem die abhäsive Ausrüstung der

Abdeckschicht unter Vermeidung einer Silikonisierung vorgenommen ist.

Die Lösung dieser Aufgabe, insbesondere die Vereinigung von hinreichender Stabilität bei gleichzeitig ausreichender Oberflächenhärte und Flexibilität erwies sich als schwierig, da diese Eigenschaften einander zuwiderlaufen. Erreicht man ein stabiles Laminat, so weist dieses aufgrund hohen Weichmacherzusatzes keine ausreichende Oberflächenhärte auf. Bei genügender Oberflächenhärte wird das Laminat dagegen bei Lagerung so schnell spröde, daß eine Applikation aufgrund der Sprödigkeit des Laminats nicht mehr möglich war.

Insbesondere mußte für eine problemlose Anformbarkeit eine Formulierung gefunden werden, , wobei die Schutzschicht gegenüber den übrigen Bestandteilen des Laminats und den bei der Herstellung des Laminats verwendeten Materialien resistent und die Adhäsion der Abdeckschicht (Schutzschicht) an der Polymerschicht durch eine abhäsive Ausrüstung unter Vermeidung einer Silikonisierung auf der beschichteten Seite geringer als die Adhäsion zwischen der Haftkleberschicht und der Schutzschicht (Trägerfolie) ist.

Überraschenderweise wurde gefunden, daß die Kombination dieser für die Anwendung wichtigen Eigenschaften dadurch erreicht wird, daß die abhäsive Ausrüstung durch eine Beschichtung erreicht wird, die als wesentliches Bestandteil ein Komplexsalz aus einer Fettsäure von 12 bis 22 Kohlenstoffatomen, die gesättigt ist oder bis zu 3 olefinische Bindungen enthält, und ein mindestens zweiwertiges Metall enthält. Dieses Material hat den Vorteil, daß es eine absolut glatte Oberfläche ergibt, sodaß die Polymerschicht Hochglanz aufweist.

Eine Ausgestaltung der Erfindung sieht dabei mit Vorteil vor, daß das mindestens zweiwertige Metall ein Übergangsmetall ist. Es kann mit Vorteil das Schwermetall Chrom sein.

Gegenstand der Erfindung ist somit ein selbstklebendes, an Fuß und Fingernägel anformbares Laminat, enthaltend die Komponenten a) bis f) gemäß dem Oberbegriff von Anspruch 1 mit einer die Adhäsion der Abdeckschicht (Schutzschicht) an der Polymerschicht durch eine erfindungsgemäß Ausrüstung mit einer abhäsiven Beschichtung unter Vermeidung einer Silikonisierung, entsprechend den kennzeichnenden Merkmalen von Anspruch 1.

Die Beschichtung des Lackes (d.h. der Polymerschicht) auf die vor der Applikation zu entfernende Abdeckschicht ermöglicht einerseits eine Strukturierung der Oberfläche der Polymerschicht und andererseits stellt sie die Stabilität und Gebrauchsfähigkeit des Laminats bis zur Applikation sicher. Die oberflächige Strukturierung der Polymerschicht entspricht einem Negativ-Abdruck.

Die Schurzschicht d) entspricht in ihrer Größe mindestens der Größe des Laminats und ist vorzugsweise größer. Sie kann aus einer Folie, vorzugsweise einer Kunststoffolie, einem Papier oder aus einem textilen Flächengebilde bestehen. Vorteilhaft besteht sie aus einem transparenten Material, um Verwechslungen hinsichtlich der Farbe beim Auftragen zu vermeiden. Sie kann ein- oder mehrschichtig ausgebildet sein, wobei die Schichten eine unterschiedliche Zusammensetzung haben können. Beispielsweise kann sie aus einer mit einem der nachstehend genannten Kunststoffe beschichteten Papierfolie bestehen.

Als Kunststoffolien eignen sich z.B. solche aus Polyestern, wie Polyethylen-terephthalat, Polybutylenterephthalat oder Polyethylen-sebacat, aus Polyethylen, Polypropylen oder Polyamiden wie Polyhexamethylenadipat, Polycaprolactam oder Poly(w-undecansäureamid). Diese Kunststoffe sind naturgemäß aufgrund ihrer Oberflächeneigenschaften noch nicht wieder ablösbar. Um ihnen diese Eigenschaft zu vermitteln, ist eine Oberflächenbehandlung mit geeigneten Stoffen, im Rahmen der vorliegenden Erfindung mit Salzen aus langkettigen Fettsäuren von z.B. 12 bis 22 Kohlenstoffatomen, die gesättigt sind oder bis zu drei olefinische Bindungen enthalten können, und mindestens zweiwertigen Metallen, insbesondere solchen von Übergangsschwermetallen, und hiervon vor allem des Chroms, vorgesehen.

Ein textiles Flächengebilde kann Vliesstoff oder Gewebe sein, die zweckmäßig abhäsiv ausgerüstet sind.

Die Schutzschicht d) ist auf der mit der Polymerschicht a) beschichteten Seite bevorzugt abhäsiv ausgerüstet, d.h. die Adhäsion zwischen ihr und dem Laminat ist geringer als die Adhäsion zwischen Kleberschicht und Trägerfolie. Vorteilhaft wird die Trägerfolie mit einer Abziehhilfe versehen, z.B. indem in ihr halbrunde Einschnitte angebracht werden. Dadurch wird die Handhabung erleichtert.

Die Kleberschicht (b) des Laminats kann aus einer bekannten Klebemasse bestehen und ebenfalls ein- oder mehrschichtig sein. Das Flächengewicht der Kleberschicht liegt im allgemeinen im Bereich von 20 bis 100 g/ $m^2$, vorzugsweise bei 25 bis 75 g/$m^2$.

Die Polymerschicht des Laminats, die ein- oder ebenfalls mehrschichtig sein kann, weist im allgemeinen eine Schichtdicke von 25 bis 200, vorzugsweise 60 bis 150 $\mu$m auf, wobei bei mehrschichtiger Ausbildung die einzelnen Schichten eine unterschiedliche Zusammensetzung aufweisen können.

Die Konzentration der Weichmacher in der Polymerschicht (auch die Kleberschicht kann den gleichen oder einen anderen Weichmacher enthalten) beträgt 15 bis 25, vorzugsweise 17 bis 22 Gew.-%, bezogen auf die Polymeren.

Als Weichmacher werden bevorzugt solche verwendet, deren Wanderung nur äußerst gering ist, wie übliche Citronensäureester oder Dioctyladipat. Gegebenenfalls kann Campher als zusätzlicher Weichmacher verwendet werden, was jedoch nicht bevorzugt ist. In diesem Fall beträgt die bevorzugte Weichmachermenge 5 bis 12,5, insbesondere 7 bis 10 Gew.-%. Da die Polymerschicht a) vielfach aus üblichen Nagellak-

ken hergestellt ist, enthält sie häufig auch noch andere weichmachende Verbindungen wie Phthalate, z.B. Dibutylphthalat.

Die Polymerschicht besteht vorteilhaft wenigstens zum Teil aus Nitrocellulose allein oder Acrylaten allein oder Gemischen dieser beiden an sich für Nagellacke bekannten Komponenten. Als Acrylate werden dabei insbesondere Polymere von Acryl- bzw. Methacrylsäureestern verwendet, vor allem von Alkoholen mit 1 bis 4, vorzugsweise 1 bis 2 C-Atomen, und/oder Dimethylaminoethylmethacrylat. Die Polymerschicht enthält meistens auch noch wenigstens ein in der Kosmetikindustrie übliches Farbmittel, also Farbstoffe und/oder Pigmente; jedoch ist dieses nicht notwendig. Die erfindungsgemäßen selbstklebenden Laminate können nämlich auch transparent sein, wenn dieses gewünscht wird.

Der Polymerschicht a) und/oder der Kleberschicht b) können Zusätze wie geruchsaktive Komponenten und/oder Wirkstoffe, vorzugsweise antimykotisch wirksame Stoffe wie Clotrimazol, Miconazol, Ketoconazol, Econazol, Naftidin, Ciclopiroxolamin, Fenticlor, Sulbentin, Tolnaftat und Haloprogin zugesetzt werden. In solchen Fällen besteht die Kleberschicht vorteilhaft aus mehreren Schichten.

Gemäß der Erfindung können als Wirkstoffe auch Verbindungen der Formel

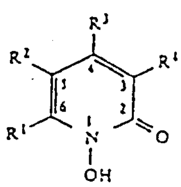

verwendet werden, wie sie in dem EP 226 984 (= US-PS 4 957 730) als Bestandteile in Nagellacken mit dem Ziel der Behandlung von Onychomnikosen beschrieben sind. Nach der genannten Patentschrift werden diese Nagellacke in flüssiger Form aufgetragen. In den genannten Verbindungen bedeutet $R^1$ eine Kohlenwasserstoffgruppe, die 6 bis 9 Kohlenstoffatome enthält, jedoch von olefinischen und acetylenischen Bindungen frei ist; eine der Gruppen $R^2$ und $R^4$ bedeutet Wasserstoff und die andere Wasserstoff, Methyl oder Ethyl, und $R^3$ ist Alkyl mit bis zu 2 C-Atomen; diese Wirkstoffe können in freier Form oder in Form eines Salzes zugegen sein. $R^1$ kann z.B. eine Cyclohexyl- oder octylgruppe, z.B. der Formel $CH_2 - CH(CH_3)\text{-}CH_2\text{-}C(CH_3)_3$ sein. Die Einarbeitung der genannten Verbindungen in die erfindungsgemäßen Laminate hat gegenüber den Lacken gemäß der EP 226 954 die Vorteile, daß die Bioverfügbarkeit des Wirkstoffes aus dem Laminat durch die standardisierte Schichtdicke in dem jeweils gewünschten Ausmaß gesichert ist und daß die Einwirkungsdauer durch die Dicke des Laminats nach Belieben eingestellt werden kann. Wie durch In-Vitro-Versuche belegt werden konnte, ist die Kleberschicht des erfindungsgemäßen Laminats für die genannten Wirkstoffe durchlässig, stellt also keine Barriere für sie dar, wenn sich diese Wirkstoffe in der Polymerschicht befinden. Vielmehr kann man die Einwirkung des Wirkstoffs auf den Nagel noch beschleunigen, wenn man den Wirkstoff auch in die Kleberschicht einarbeitet.

Die Kleberschicht kann ferner eine Substanz enthalten, die die Penetration des Wirkstoffs in den Nagel fördert. Der Zusatz von Penetrationsbeschleunigern bzw. -verstärkern (Enhancern) zu einer Wirkstoff/Kleber-Rezeptur ist aus dem Bereich der transdermalen therapeutischen Systeme bekannt. Substanzen, die einen Wirkstofftransport in die Haut bzw. durch die Haut fördern, sind z.B. Dimethylsulfoxid (DMSO) und Dimethylformamid (DMF) wie auch eine große Anzahl verschiedener Emulgatoren, Fettsäuren und deren Ester.

Die Erfindung weist die folgenden Vorteile auf: Es wird kein organisches Lösungsmittel an die Umwelt abgegeben. Die erfindungsgemäßen, als künstliche Fuß- oder Fingernägel geeigneten Laminate erfüllen alle vom Verbraucher an sie gestellten Anforderungen hinsichtlich Ganz, Härte, Flexibilität und Haltbarkeit. Ihre Eigenschaften sind so, daß eine Verletzung des Nagelbettes bei Anwendung und Gebrauch vermieden wird. Darüber hinaus kann die Polymerschicht durch die Verwendung einer geeigneten strukturierten Abdeckschicht auch für dekorative Zwecke strukturiert sein. Schließlich betrifft die Erfindung auch die Verwendung der erfindungsgemäßen Laminate zur Aufbringung auf Fuß- oder Fingernägel als künstliche Fuß- oder Fingernägel.

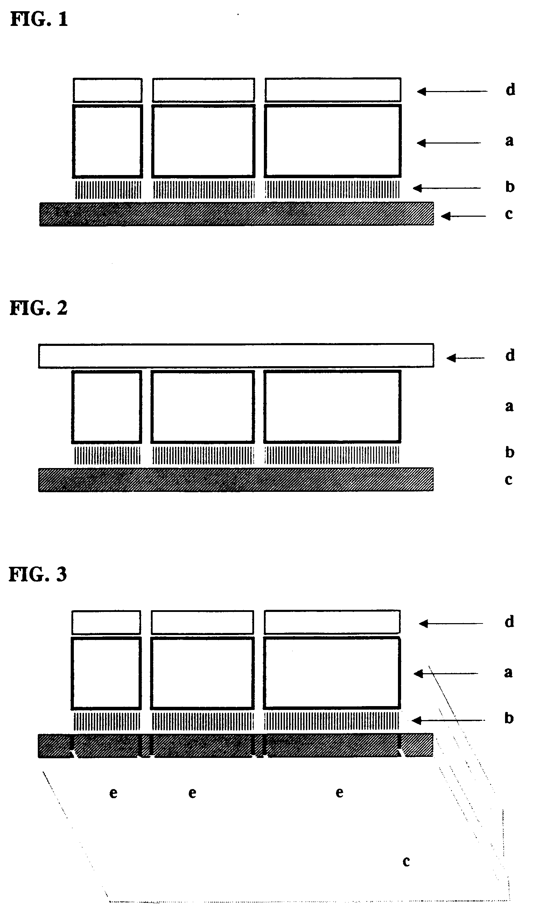

Die Erfindung wird anhand der Figuren 1 bis 3 und durch die nachfolgenden Beispiele in nicht einschränkender Weise erläutert:

Figur 1 stellt eine Karte mit drei Bereichen im Querschnitt dar, wie sie im Beispiel 1 beschrieben wird. Auf einer Schutzschicht d) (Polyesterfolie einer Dicke von 100 µm, die eine zum Teil strukturierte Oberfläche hat und vor der Applikation zu entfernen ist) ist eine Polymerschicht a) (Nagellack pigmentiert, Schichtdicke z.B. 200 µm) und auf der Polymerschicht a) eine Kleberschicht b) (auf der Basis von Polyisobutylen, Schichtdicke z.B. 80 µm) angeordnet. Das Laminat ist mit einer Trägerfolie c) ausgerüstet.

Figur 2 zeigt ein Laminat im Querschnitt, wo die Schutzschicht d) übergreifend mehrere Bereiche abdeckt.

Figur 3 zeigt die Aufsicht auf einen Bereich, wobei die Trägerfolie c) die Abziehhilfe e) aufweist.

Beispiel 1:

a) Polymerschicht - Eine im Handel erhältliche Nagellackmasse bestehend aus

| | |
|---|---|
| 136,5 g | Nitrocellulose (als Hart-Polymer) |
| 50,0 g | Polyacrylester ([R] Acronal 2F der BASF AG, Ludwigshafen, als Weichpolymer) |
| 50,0 g | Toluolsulfonamidharz ([R] Santolite MHP der Monsanto. USA) |
| 23,0 g | Weichmacher (Citronensäureester, [R] Citroflex A der Pfizer, USA) |
| 60,0 g | Dibutylphthalat |
| 210 g | Ethylacetat |
| 200 g | Toluol |
| 100 g | Butylacetat |
| 77 g | Ethanol |
| 73,5g | n-Butanol |
| 20 g | handelsübliches, in der Kosmetikindustrie eingesetztes Farbpigment |
| 1000 g | |

wird auf eine erfindungsgemäß abhäsiv ausgerüstete Schutzschicht d) (Polyethylenterephthalatfolie 50 µm) aufgetragen und getrocknet (Dicke der Polymerschicht 200 µm).

b) Haftkleberschicht - Eine Klebemasse wird aus folgenden Bestandteilen hergestellt:

| | |
|---|---|
| 0,153 kg | Polyisobutylen (mittleres Molekulargewicht von 900.000 bis 1.400.000 - [R] Oppanol B 100 der BASF AG, Ludwigshafen) |
| 0,137 kg | festes aliphatisches Kohlenwasserstoffharz (Fp 100° C - [R] Hercures C der Hercules, Wilmington, Del. USA) |
| 0,137 kg | flüssiges hydriertes Kolophoniumharz ([R] Abitol der Hercules, Wilmington, Del. USA) |
| 0,005 kg | Stabilisator (Antioxidans) |
| 1,148 kg | Spezialbenzin (Kp 80 - 110° C) als Lösungsmittel |

Diese Masse wird auf eine einseitig abhäsiv ausgestattete Trägerfolie (Polyesterfolie) aufgetragen und getrocknet, (Kleberschichtdicke 80 µm).

In das so erhaltene Laminat werden durch die Schutzschicht d), die Polymerschicht a) und die Haftkleberschicht b) Bereiche eingestanzt, ohne die Trägerfolie c) zu durchstanzen. Die Ausmaße der Bereiche entsprechen in etwa denen unterschiedlicher Fingernägel. Die Zwischenstege zwischen den Bereichen werden entfernt. Das so erhaltene Laminat kann durch Schneiden derart zerteilt werden, daß man Laminat-Karten, nachfolgend als Karten bezeichnet, erhält, die mindestens 10 Bereiche aufweisen, und zwar je einen Bereich für einen Fingernagel von zwei Händen.

Das so hergestellte Laminat erwies sich bei einer Lagerung von 6 Monaten bei 50°C als stabil und erfüllte die geforderten Applikations- und Trageeigenschaften. Produkte ohne Schutzschicht d) wurden schon nach Lagerung von 2 Tagen bei 50°C spröde und ließen sich nicht mehr aufbringen.

Die so hergestellten Laminate werden wie folgt auf den Nagel aufgebracht:

Zur Behandlung eines Nagels wird ein Bereich mittels der Abziehhilfe von der Trägerfolie c) abgelöst und auf dem Nagel aufgeklebt. Anschließend bzw. gleichzeitig mit dem Aufkleben wird die Schutzschicht d) entfernt und dann die selbstklebende Polymerschicht a) auf dem Nagel angeformt.

Wenn die Schutzschicht d) mehrere Bereiche zusammenhängend überdeckt, kann sie für die Entnahme eines Bereiches der selbstklebenden Polymerschicht von der Trägerfolie c) abgelöst werden.

Die mit Haftkleber ausgerüstete Polymerschicht weist nach dem Aufbringen die gleichen Eigenschaften auf wie eine Lackschicht, die durch eine lösungsmittelhaltige Nagellackmasse aufgetragen worden ist, wobei jedoch im Gegensatz zum Aufkleben sogenannter künstlicher Fingernägel mit dem erfindungsgemäßen Produkt kein Fremdkörpergefühl auftritt.

Die mit dem Haftkleber ausgerüstete Polymerschicht läßt sich von einem Nagel wieder abziehen, indem man z.B. eine selbsthaftende Folie auf die Polymerschicht überstehend aufklebt und nach Andrücken der Folie an den Rändern der Polymerschicht diese insgesamt vom Nagel abzieht, also in ähnlicher Weise wie man Preisetiketten auf Verpackungen mittels eines Selbstklebebandes entfernen kann. Auf dem Nagel verbleibt kein Rest, so daß eine Reinigung des Nagels mit einem organischen Lösungsmittel unnötig ist. Dies ist ein bedeutender Vorteil bei der Anwendung der erfindungsgemäßen Laminate. Durch dieses neue Applikationssystem wird auch die bei Nagellacken oft auftretende Erscheinung des sogenannten "staining", d.i. die Penetration von Farbstoff in den Nagel, verhindert, was einen weiteren bedeutenden Vorteil darstellt.

Beispiel 2:

Beispiel 1 wird dahingehend abgewandelt, daß eine Haftklebemasse b) verwendet wird, die aus 2,08 kg einer 40%igen Lösung eines selbstvernetzenden Acrylatcopolymeren aus 2-Ethylhexylacrylat, Vinylacetat, Acrylsäure und Titanchelatester in einer Mischung in Essigsäureethylester, Ethanol, Hexan und Methanol. 147 g eines Acrylharzes aus Dimethylaminoethylmethacrylat und neutralem Methacrylsäureester sowie 20 g eines gemischt sauren Triglycerids der fraktionierten Kokosfettsäuren $C_8$ - $C_{10}$ besteht.

Beispiel 3:

Beispiel 1 wird dahingehend präzisiert, daß die dort genannte abhäsiv ausgerüstete Polymerschicht auf eine mit 1 bis 2 g/m$^2$ eines Chromstearatkomplexes ([R] Quilon C der Firma du Pont, Wilmington, Del. USA) behandelte Schutzschicht d) aus Polyethylenterephthalatfolie von 50 µm Dicke aufgetragen und getrocknet wird (Dicke der Polymerschicht ebenfalls 200 µm). Das mit diesem Produkt hergestellte Laminat hat im Vergleich zu Laminaten, die mit einer silikonisierten Folie hergestellt worden sind, den Vorteil, daß sich die Schutzschicht anschließend oder auch gleichzeitig mit dem Aufkleben auf den Nagel noch leichter entfernen läßt.

## Patentansprüche

### Patentansprüche für folgende Vertragsstaaten : AT, BE, CH, DE, DK, ES, FR, GB, GR, IT, LI, LU, NL, SE

1. Selbstklebendes, an Fuß- und Fingernägel anformbares Laminat, enthaltend

   a) eine filmbildende Polymerschicht mit 1-30% eines Weichmachers, bezogen auf die Polymeren,
   b) eine darauf befindliche Haftkleberschicht,
   c) eine die Haftkleberschicht aodeckende, wieder ablösbare Trägerfolie;
   d) eine ebenfalls ablösbare, die filmbildende Polymerschicht a) mindestens vollflächig abdeckende Schutzschicht, wobei
   e) die Schutzschicht gegenüber den übrigen Bestandteilen des Laminats und den bei der Herstellung des Laminats verwendeten Materialien resistent ist und
   f) die Adhäsion der Schutzschicht d) an der Polymerschicht a) durch eine abhäsive Ausrüstung auf der beschichteten Seite geringer als die Adhäsion zwischen der Haftkleberschicht b) und der Trägerfolie c) ist, dadurch gekennzeichnet,
   g) daß die abhäsive Ausrüstung durch eine Beschichtung erreicht wird, die als wesentliches Bestandteil ein Komplexsalz aus einer Fettsäure von 12 bis 22 Kohlenstoffatomen, die gesättigt ist oder bis zu 3 olefinische Bindungen enthält, und ein mindestens zweiwertiges Metall enthält.

2. Selbstklebendes Laminat nach Anspruch 1, dadurch gekennzeichnet, daß das mindestens zweiwertige Metall ein Übergangsmetall ist.

3. Selbstklebendes Laminat nach Anspruch 2, dadurch gekennzeichnet, daß das Schwermetall Chrom ist.

4. Selbstklebendes Laminat nach Anspruch 1, dadurch gekennzeichnet, daß die Schutzschicht d) strukturiert ist.

5. Selbstklebendes Laminat nach Anspruch 1 dadurch gekennzeichnet, daß die Schutzschicht d) aus einer Folie, vorzugsweise aus Kunststoff, einem Papier oder aus einem textilen Flächengebilde besteht.

6. Selbstklebendes Laminat nach einem oder mehreren der Ansprüche 1 bis 3, dadurch gekennzeichnet, daß die Adhäsion der Schutzschicht d) an der Polymerschicht a) geringer ist als die Adhäsion zwischen Kleberschicht b) und Trägerfolie c).

7. Selbstklebendes Laminat nach einem oder mehreren der Ansprüche 1 bis 6, dadurch gekennzeichnet, daß die Schutzschicht d) transparent ist.

8. Selbstklebendes Laminat nach einem oder mehreren der Ansprüche 1 bis 7, dadurch gekennzeichnet, daß die Kleberschicht b) und/ oder die Schutzschicht d) mehrschichtig ausgebildet sind.

9. Selbstklebendes Laminat nach einem oder mehreren der Ansprüche 1 bis 8, dadurch gekennzeichnet, daß die Trägerfolie c) mit einer Abziehhilfe versehen ist.

10. Selbstklebendes Laminat, nach einem oder mehreren der Ansprüche 1 bis 9, dadurch gekennzeichnet, daß der Haftkleber ein Flächengewicht von 20 bis 100 $g/m^2$ , vorzugsweise 25 bis 75 $g/m^2$ hat.

11. Selbstklebendes Laminat nach einem oder mehreren der Ansprüche 1 bis 10, dadurch gekennzeichnet, daß die Polymerschicht a) eine Schichtdicke von 25 bis 200 µm, vorzugsweise 60 bis 150 µm aufweist.

12. Selbstklebendes Laminat nach einem oder mehreren der Ansprüche 1 bis 11, dadurch gekennzeichnet, daß die Konzentration der Weichmacher in der Polymerschicht a) 15 bis 25 Gew.-% vorzugweise 17 bis 22 Gew.-% ist, bezogen auf die Polymeren.

13. Selbstklebendes Laminat nach einem oder mehreren der Ansprüche 1-12, dadurch gekennzeichnet, daß es einen Weichmacher enthält dessen Wanderung nur äußerst gering ist.

14. Selbstklebendes Laminat nach einem oder mehreren der Ansprüche 1 bis 12, dadurch gekennzeichnet, daß es als Weichmacher Citronensäureester enthält.

15. Selbstklebendes Laminat nach einem oder mehreren der Ansprüche 1 bis 13, dadurch gekennzeichnet, daß die Polymerschicht a) mehrschichtig ausgebildet ist.

16. Selbstklebendes Laminat nach einem oder mehreren der Ansprüche 1 bis 15, dadurch gekennzeichnet, daß die Schichten unterschiedlich zusammengesetzt sind.

**17.** Selbstklebendes Laminat nach einem oder mehreren der Ansprüche 1 bis 16, dadurch gekennzeichnet, daß die Polymerschicht a) wenigstens zum Teil aus Nitrocellulose und/oder Acrylaten besteht, wobei es sich bei den Acrylaten um Polymere von Acryl- bzw. Methacrylsäureestern, insbesondere von Alkoholen mit 1 bis 4, vorzugsweise 1 bis 2 C-Atomen, und/oder Dimethylaminoethylmethacrylat handelt.

**18.** Selbstklebendes Laminat nach einem oder mehreren der Ansprüche 1 bis 17, dadurch gekennzeichnet, daß die Polymerschicht a) aus einem handelsüblichen Nagellack gebildet ist, der frei von Campher ist.

**19.** Selbstklebendes Laminat nach einem oder mehreren der Ansprüche 1 bis 18, dadurch gekennzeichnet, daß die Polymerschicht a) und/oder die Kleberschicht b) eine geruchsaktive Komponente enthält.

**20.** Selbstklebendes Laminat nach einem oder mehreren der Ansprüche 1 bis 19, dadurch gekennzeichnet, daß die Polymerschicht a) und/oder die Kleberschicht b) einen Wirkstoff enthält.

**21.** Selbstklebendes Laminat nach einem oder mehreren der Ansprüche 1 bis 20, dadurch gekennzeichnet, daß die Polymerschicht a) und/oder die Kleberschicht b) antimykotisch wirksame Stoffe enthält.

**22.** Selbstklebendes Laminat nach Anspruch 20, dadurch cekennzeichnen daß die Polymerschicht a) und/oder die Kleberschicht b) als antimykotische Wirkstoffe Clotrimazol, Miconazol, Ketoconazol, Econazol, Naftidin, Fenticlor, Sulbentin, Tolnaftat, und/oder Haloprogin oder als antimykotische Verbindung ein 1-Hydroxy-2-Pyridon der Formel

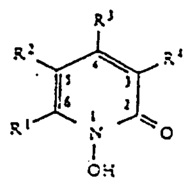

enthält, in der bedeuten $R^1$ eine Kohlenwasserstoffgruppe, die 6 bis 9 Kohlenstoffatome enthält, jedoch von olefinischen und acetylenischen Bindungen frei ist, eine der Gruppen $R^2$ und $R^4$ Wasserstoff und die andere Wasserstoff, Methyl oder Ethyl und $R^3$ Alkyl mit bis zu 2 C-Atomen.

**23.** Selbstklebendes Laminat nach einem oder mehreren der Ansprüche 1 bis 22, dadurch gekennzeichnet, daß die Kleberschicht b) einen die Penetration des Wirkstoffes in den Nagel fördernden Anteil enthält, wie Dimethylsulfoxid oder Dimeth ylformamid.

**24.** Selbstklebendes Laminat nach einem oder mehreren der Ansprüche 1 bis 23, dadurch gekennzeichnet, daß die Schutzschicht d) auf der beschichteten Seite zuvor abhäsiv ausgerüstet ist.

**25.** Selbstklebendes Laminat nach einem oder mehreren der Ansprüche 1 bis 24, dadurch gekennzeichnet, daß die Polymerschicht ein Farbmittel, einen Farbstoff, ein Pigment oder eine Kombination davon enthält.

**26.** Verwendung des Laminats nach einem oder mehreren der Ansprüche 1 bis 25 zur Aufbringung auf Fuß- oder oder Fingernägel als künstliche Nägel.

**Patentansprüche für folgende Vertragsstaaten : IE, MC, PT**

**1.** Selbstklebendes, an Fuß- und Fingernägel anformbares Laminat, enthaltend

a) eine filmbildende Polymerschicht,
b) eine darauf befindliche Haftkleberschicht und
c) eine die Haftkleberschicht abdeckende, wieder ablösbare Trägerfolie sowie
d) eine ebenfalls ablösbare, die filmbildende Polymerschicht a) mindestens vollflächig abdeckende Schutzschicht, gekennzeichnet durch die Merkmale:
e) die filmbildende Polymerschicht enthält 1 bis 30 Gew.-% eines Weichmachers, bezogen auf die Polymeren,
f) die Schutzschicht ist gegenüber den übrigen Bestandteilen des Laminats und den bei der Herstellung des Laminats verwendeten Materialien resistent,
g) die Adhäsion der Schutzschicht d) an der Polymerschicht a) ist geringer als die Adhäsion zwischen Kleberschicht b) und Trägerfolie c).

**2.** Selbstklebdes Laminat nach Anspruch 1, dadurch gekennzeichnet, daß die Schutzschicht d) strukturiert ist.

**3.** Selbstklebendes Laminat nach Anspruch 1 oder 2, dadurch gekennzeichnet, daß die Schutzschicht d) aus einer Folie, vorzugsweise aus Kunststoff, einem Papier oder aus einem textilen Flächengebilde besteht.

**4.** Selbstklebendes Laminat nach einem oder mehreren der Ansprüche 1 bis 3, dadurch gekennzeichnet, daß die Schutzschicht d) transparent ist.

**5.** Selbstklebendes Laminat nach einem oder mehreren der Ansprüche 1 bis 4, dadurch gekennzeichnet, daß die Kleberschicht b) und/ oder die Schutzschicht d) mehrschichtig ausgebildet sind.

6. Selbstklebendes Laminat nach einem oder mehreren der Ansprüche 1 bis 5, dadurch gekennzeichnet, daß die Trägerfolie c) mit einer Abziehhilfe versehen ist.

7. Selbstklebendes Laminat, nach einem oder mehreren der Ansprüche 1 bis 6, dadurch gekennzeichnet, daß der Haftkleber ein Flächengewicht von 20 bis 100 $g/m^2$ , vorzugsweise 25 bis 75 $g/m^2$ hat.

8. Selbstklebendes Laminat nach einem oder mehreren der Ansprüche 1 bis 7, dadurch gekennzeichnet, daß die Polymerschicht a) eine Schichtdicke von 25 bis 200 µm, vorzugsweise 60 bis 150 µm aufweist.

9. Selbstklebendes Laminat nach einem oder mehreren der Ansprüche 1 bis 8, dadurch gekennzeichnet, daß Konzentration der Weichmacher in der Polymerschicht a) 15 bis 25 Gew.-% und insbesondere 17 bis 22 Gew.-% ist, bezogen auf die Polymeren.

10. Selbstklebendes Laminat nach einem oder mehreren der Ansprüche 1 bis 9, dadurch gekennzeichnet, daß es einen Weichmacher enthält, dessen Wanderung nur äußerst gering ist.

11. Selbstklebendes Laminat nach einem oder mehreren der Ansprüche 1 bis 10, dadurch gekennzeichnet, daß es als Weichmacher Citronensäureester enthält.

12. Selbstklebendes Laminat nach einem oder mehreren der Ansprüche 1 bis 11, dadurch gekennzeichnet, daß die Polymerschicht a) mehrschichtig ausgebildet ist.

13. Selbstklebendes Laminat nach einem oder mehreren der Ansprüche 6 bis 12, dadurch gekennzeichnet, daß die Schichten unterschiedlich zusammengesetzt sind.

14. Selbstklebendes Laminat nach einem oder mehreren der Ansprüche 1 bis 13, dadurch gekennzeichnet, daß die Polymerschicht a) wenigstens zum Teil aus Nitrocellulose und/oder Acrylaten besteht, wobei es sich bei den Acrylaten um Polymere von Acryl- bzw. Methacrylsäureestern, insbesondere von Alkoholen mit 1 bis 4, vorzugsweise 1 bis 2 C-Atomen, und/oder Dimethylaminoethylmethacrylat handelt.

15. Selbstklebendes Laminat nach einem oder mehreren der Ansprüche 1 bis 14, dadurch gekennzeichnet, daß die Polymerschicht a) aus einem handelsüblichen Nagellack gebildet ist, der frei von Campher ist.

16. Selbstklebendes Laminat nach einem oder mehreren der Ansprüche 1 bis 15, dadurch gekennzeichnet, daß die Polymerschicht a) und/oder die Kleberschicht b) eine geruchsaktive Komponente enthält.

17. Selbstklebendes Laminat nach einem oder mehreren der Ansprüche 1 bis 16, dadurch gekennzeichnet, daß die Polymerschicht a) und/oder die Kleberschicht b) einen Wirkstoff enthält.

18. Selbstklebendes Laminat nach einem oder mehreren der Ansprüche 1 bis 17, dadurch gekennzeichnet, daß die Polymerschicht a) und/oder die Kleberschicht b) antimykotisch wirksame Stoffe enthält.

19. Selbstklebendes Laminat nach Anspruch 18, dadurch cekennzeichnen daß die Polymerschicht a) und/oder die Kleberschicht b) als antimykotische Wirkstoffe Clotrimazol, Miconazol, Ketoconazol, Econazol, Naftidin, Fenticlor, Sulbentin, Tolnaftat, und/oder Haloprogin oder als antimykotische Verbindung ein 1-Hydroxy-2-Pyridon der Formel

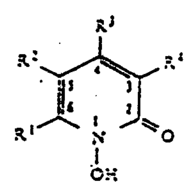

enthält, in der bedeuten $R^1$ eine Kohlenwasserstoffgruppe, die 6 bis 9 Kohlenstoffatome enthält, jedoch von olefinischen und acetylenischen Bindungen frei ist, eine der Gruppen $R^2$ und $R^4$ Wasserstoff und die andere Wasserstoff, Methyl oder Ethyl und $R^3$ Alkyl mit bis zu 2 C-Atomen.

20. Selbstklebendes Laminat nach einem oder mehreren der Ansprüche 1 bis 19, dadurch gekennzeichnet, daß die Kleberschicht b) einen die Penetration des Wirkstoffes in den Nagel fördernden Anteil enthält, wie Dimethylsulfoxid oder Dimeth ylformamid.

21. Selbstklebendes Laminat nach einem oder mehreren der Ansprüche 1 bis 20, dadurch gekennzeichnet, daß die Schutzschicht d) auf der beschichteten Seite zuvor abhäsiv ausgerüstet ist.

22. Selbstklebendes Laminat nach Anspruch 21, dadurch gekennzeichnet, daß die abhäsive Ausrüstung durch eine Beschichtung erreicht wird, die als wesentlichen Bestandteil ein Komplexsalz aus

einer Fettsäure von 12 bis 22 Kohlenstoffatomen, die gesöttigt ist oder bis zu 3 olefinische Bindungen enthält, und einem mindestens zweiwertigen Metall enthält.

**23.** Selbstklebendes Laminat nach Anspruch 22, dadurch gekennzeichnet, daß das mindestens zweiwertige Metall ein Übergangsschwermetall ist.

**24.** Selbstklebendes Laminat nach Anspruch 23, dadurch gekennzeichnet, daß das Schwermetall Chrom ist.

**25.** Selbstklebendes Laminat nach Anspruch 21, dadurch gekennzeichnet, daß die abhäsive Ausrüstung durch eine Beschichtung mit einem hierfür geeigneten Silikon erreicht wird.

**26.** Selbstklebendes Laminat nach einem oder mehreren der Ansprüche 1 bis 25, dadurch gekennzeichnet, daß das Farbmittel ein Farbstoff oder ein Pigment oder eine Kombination davon ist.

**27.** Verfahren zur Herstellung eines selbstklebenden, an Fuß oder Fingernägel anformbaren Laminats nach einem oder mehreren der Ansprüche 1 bis 26, dadurch gekennzeichnet, daß man einen Lack, der wenigstens einen Weichmacher und wenigstens ein filmbildendes Polymer, gelöst oder dispergiert in einem organischen Lösungsmittel, und vorteilhaft auch ein Farbmittel enthält, auf eine abziehbare Schutzschicht d) aufbringt, die wenigstens die gleiche Größe wie das Laminat hat, den Lack unter Rückgewinnung des Lösungsmittels trocknet und die mit dem Lack beschichtete Schutzschicht d) lackseitig mit einem Kleberfilm und einer Trägerfolie c) laminiert.

**28.** Verwendung des Laminats nach einem oder mehreren der Ansprüche 1 bis 26 bzw'. des nach dem Verfahren des Anspruchs 27 erhaltenen Laminats zur Aufbringung auf Fuß- oder Fingernägel als künstliche Nägel.

**Claims**

**Claims for the following Contracting States : AT, BE, CH, DE, DK, ES, FR, GB, GR, IT, LI, LU, NL, SE**

**1.** A self-adhesive laminate formable to toenails and fingernails, containing

a) a film-forming polymeric layer comprising 1 to 30%-wt of a plasticizer, relative to the polymers,
b) a pressure-sensitive adhesive layer located thereon,
c) a removable carrier sheet covering the pressure-sensitive adhesive layer,
d) a likewise removable protective layer covering at least the entire area of the film-forming polymeric layer a),

wherein

e) the protective layer is resistent to the other components of the laminate and to the materials employed in the production of the laminate,
f) due to an abhesive finish on the coated side, the adhesion of the protective layer d) to the polymer layer a) is less than the adhesion between adhesive layer b) and carrier sheet c)

characterized in that

g) the abhesive finish is achieved by a coating containing as essential component a complex salt of a fatty acid having 12 to 22 carbon atoms which is saturated or contains up to 3 olefinic bonds, and a metal which is at least bivalent.

**2.** The self-adhesive laminate according to claim 1 characterized in that the metal which is at least bivalent is a transition heavy metal.

**3.** The self-adhesive laminate according to claim 2 characterized in that the heavy metal is chromium.

**4.** The self-adhesive laminate according to claim 1 characterized in that the protective layer d) has a structure.

**5.** The self-adhesive laminate according to claim 1 characterized in that the protective layer d) consists of a sheet, preferably made of plastics material, of a paper, or of a textile fabric.

**6.** The self-adhesive laminate according to one or more of claims 1 to 3 characterized in that the adhesion of the protective layer d) to the polymer layer a) is less than the adhesion between adhesive layer b) and carrier sheet c).

**7.** The self-adhesive laminate according to one or more of claims 1 to 6 characterized in that the protective layer d) is transparent.

**8.** The self-adhesive laminate according to one or more of claims 1 to 7 characterized in that the adhesive layer b) and/or the protective layer d) have a multi-layered structure.

**9.** The self-adhesive laminate according to one or more of claims 1 to 8 characterized in that the carrier sheet c) is provided with a peel-off aid.

**10.** The self-adhesive laminate according to one or more of claims 1 to 9 characterized in that the

weight per area of the pressure-sensitive adhesive amounts to 20 to 100 g/$m^2$, preferably 25 to 75 g/$m^2$.

**11.** The self-adhesive laminate according to one or more of claims 1 to 10 characterized in that the polymeric layer a) has a layer thickness of 25 to 200 µm, preferably 60 to 150 µm.

**12.** The self-adhesive laminate according to one or more of claims 1 to 11 characterized in that the concentration of the plasticizers within the polymeric layer a) amounts to 15 to 25%-wt., preferably 17 to 22%-wt., relative to the polymers.

**13.** The self-adhesive laminate according to one or more of claims 1 to 12, characterized in that it contains a plasticizer the migration of which is extremely low.

**14.** The self-adhesive laminate according to one or more of claims 1 to 12 characterized in that it contains citric ester as plasticizer.

**15.** The self-adhesive laminate according to one or more of claims 1 to 13 characterized in that the polymeric layer a) has a multi-layered structure.

**16.** The self-adhesive laminate according to one or more of claims 1 to 15 characterized in that the layers have differing compositions.

**17.** The self-adhesive laminate according to one or more of claims 1 to 16 characterized in that the polymeric layer a) at least partially consists of nitrocellulose and/or acrylates, the acrylates being polymers of acrylic or methacrylic acid esters, especially of alcohols having 1 to 4, prefererably 1 to 2 C atoms, and/or dimethylaminoethyl methacrylate.

**18.** The self-adhesive laminate according to one or more of claims 1 to 17 characterized in that the polymer layer a) is made of a commercial nail lacquer which is free of camphor.

**19.** The self-adhesive laminate according to one or more of claims 1 to 18 characterized in that the polymeric layer a) and/or the adhesive layer b) contain an odour-active component.

**20.** The self-adhesive laminate according to one or more of claims 1 to 19 characterized in that the polymeric layer a) and/or the adhesive layer b) comprise an active substance.

**21.** The self-adhesive laminate according to one or more of claims 1 to 20 characterized in that the polymeric layer a) and/or the adhesive layer b) comprise substances having an antimycotic effect.

**22.** The self-adhesive laminate according to claim 21 characterized in that the polymeric layer a) and/or the adhesive layer b) comprise as antimycotic active substances, clotrimazole, miconazole, ketoconazole, econazole, naftidin, fenticlor, sulbentine, tolnaftate and/or haloprogine, or as antimycotic compounds a 1-hydroxy-2-pyridone of the formula

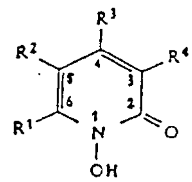

in which $R^1$ is a hydrocarbon group which contains 6 to 9 carbon atoms but is free of olefinic and acetylenic bonds, one of the groups $R^2$ and $R^4$ is hydrogen and the other one is hydrogen, methyl or ethyl, and $R^3$ is alkyl having up to 2 C atoms.

**23.** The self-adhesive laminate according to one or more of claims 1 to 22 characterized in that the adhesive layer b) comprises a proportion promoting the penetration of the active substance into the nail, such as dimethyl sulfoxide or dimethyl formamide.

**24.** The self-adhesive laminate according to one or more of claims 1 to 23 characterized in that the protective layer d) has previously been rendered abhesive on the coated side.

**25.** The self-adhesive laminate according to one or more of claims 1 to 24 characterized in that the polymer layer contains a colourant, a dye or a pigment, or a combination thereof.

**26.** The use of the laminate as defined in any one of claims 1 to 25 for applying it as artificial nails on toenails or fingernails.

**Claims for the following Contracting States : IE, MC, PT**

**1.** A self-adhesive laminate formable to toenails and fingernails, containing

a) a film-forming polymeric layer,
b) a pressure-sensitive adhesive layer located thereon,
c) a removable carrier sheet covering the pressure-sensitive adhesive layer,
d) a likewise removable protective layer covering at least the entire area of the film-forming polymeric layer a),

and characterized by the following features:

e) the film-forming polymer layer contains 1 to 30%-wt of a plasticizer, relative to the polymers,
f) the protective layer is resistent to the other components of the laminate and to the materials employed in the production of the laminate,
g) the adhesion of the protective layer d) to the polymer layer a) is lower than the adhesion between adhesive layer b) and carrier sheet c).

**2.** The self-adhesive laminate according to claim 1 characterized in that the protective layer d) has a structure.

**3.** The self-adhesive laminate according to claim 1 or 2 characterized in that the protective layer d) consists of a sheet, preferably made of plastics material, of a paper, or of a textile fabric.

**4.** The self-adhesive laminate according to one or more of claims 1 to 3 characterized in that the protective layer d) is transparent.

**5.** The self-adhesive laminate according to one or more of claims 1 to 4 characterized in that the adhesive layer b) and/or the protective layer d) have a multi-layered structure.

**6.** The self-adhesive laminate according to one or more of claims 1 to 5 characterized in that the carrier sheet c) is provided with a peel-off aid.

**7.** The self-adhesive laminate according to one or more of claims 1 to 6 characterized in that the weight per area of the pressure-sensitive adhesive amounts to 20 to 100 $g/m^2$, preferably 25 to 75 $g/m^2$.

**8.** The self-adhesive laminate according to one or more of claims 1 to 7 characterized in that the polymeric layer a) has a layer thickness of 25 to 200 $\mu m$, preferably 60 to 150 $\mu m$.

**9.** The self-adhesive laminate according to one or more of claims 1 to 8 characterized in that the concentration of the plasticizers within the polymeric layer a) amounts to 15 to 25%-wt., and in particular 17 to 22%-wt., relative to the polymers.

**10.** The self-adhesive laminate according to one or more of claims 1 to 9, characterized in that it contains a plasticizer whose migration is extremely low.

**11.** The self-adhesive laminate according to one or more of claims 1 to 10 characterized in that it contains citric ester as plasticizer.

**12.** The self-adhesive laminate according to one or more of claims 1 to 11 characterized in that the polymeric layer a) is multi-layered.

**13.** The self-adhesive laminate according to one or more of claims 6 to 12 characterized in that the layers have differing compositions.

**14.** The self-adhesive laminate according to one or more of claims 1 to 13 characterized in that the polymeric layer a) at least partially consists of nitrocellulose and/or acrylates, the acrylates being polymers of acrylic or methacrylic acid esters, especially of alcohols having 1 to 4, prefererably 1 to 2 C atoms, and/or dimethylaminoethyl methacrylate.

**15.** The self-adhesive laminate according to one or more of claims 1 to 14 characterized in that the polymer layer a) is made of a commercial nail lacquer which is free of camphor.

**16.** The self-adhesive laminate according to one or more of claims 1 to 15 characterized in that the polymeric layer a) and/or the adhesive layer b) contain an odour-active component.

**17.** The self-adhesive laminate according to one or more of claims 1 to 16 characterized in that the polymeric layer a) and/or the adhesive layer b) comprise an active substance.

**18.** The self-adhesive laminate according to one or more of claims 1 to 17 characterized in that the polymeric layer a) and/or the adhesive layer b) comprise substances having an antimycotic effect.

**19.** The self-adhesive laminate according to claim 18 characterized in that the polymeric layer a) and/or the adhesive layer b) comprise as antimycotic active substances, clotrimazole, miconazole, ketoconazole, econazole, naftidin, fenticlor, sulbentine, tolnaftate and/or haloprogine, or as antimycotic compounds a 1-hydroxy-2-pyridone of the formula

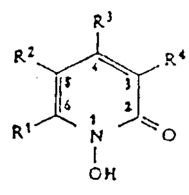

in which $R^1$ is a hydrocarbon group which contains 6 to 9 carbon atoms but is free of olefinic and acetylenic bonds, one of the groups $R^2$ and $R^4$ is hydrogen and the other one is hydrogen, methyl or ethyl, and $R^3$ is alkyl having up to 2 C atoms.

20. The self-adhesive laminate according to one or more of claims 1 to 19 characterized in that the adhesive layer b) comprises a proportion promoting the penetration of the active substance into the nail, such as dimethyl sulfoxide or dimethyl formamide.

21. The self-adhesive laminate according to one or more of claims 1 to 20 characterized in that the protective layer d) has previously been rendered abhesive on the coated side.

22. The self-adhesive laminate according to claim 21 characterized in that the abhesive finish is achieved by a coating containing as essential component a complex salt of a fatty acid having 12 to 22 carbon atoms which is saturated or contains up to 3 olefinic bonds, and a metal which is at least bivalent.

23. The self-adhesive laminate according to claim 22 characterized in that the metal, which is at least bivalent, is a transition heavy metal.

24. The self-adhesive laminate according to claim 23 characterized in that the heavy metal is chromium.

25. The self-adhesive laminate according to claim 21 characterized in that the abhesive finish is achieved by coating with a suitable silicone.

26. The self-adhesive laminate according to one or more of claims 1 to 25 characterized in that the colourant is a dye or a pigment or a combination thereof.

27. A process for the production of a self-adhesive laminate which can be shaped to toenails or fingernails as defined in any one of claims 1 to 26, characterized in that a lacquer containing at least one plasticizer and at least one film-forming polymer, dissolved or dispersed in an organic solvent, and advantageously also contains a colourant, is applied on a removable protective layer d) having at least the same size as the laminate, the lacquer is dried under solvent recovery, and the protective layer d), which is coated with the lacquer, is laminated on the lacquer-coated side with an adhesive film and a carrier sheet c).

28. Use of the laminate as defined in any one of claims 1 to 26 and of the laminate obtained according to the process of claim 27, respectively, for applying it as artificial nails on toenails or fingernails.

**Revendications**

**Revendications pour les Etats contractants suivants : AT, BE, CH, DE, DK, ES, FR, GB, GR, IT, LI, LU, NL, SE**

1. Lamifié autocollant adaptable aux ongles des pieds et/ou des doigts, qui contient :

   a) une couche de polymères filmogène avec de 1 à 30% d'un plastifiant, par rapport aux polymères,
   b) une couche de colle de contact par-dessus la couche de polymères,
   c) une feuille de support amovible, recouvrant la couche de colle de contact,
   d) une couche protectrice, également amovible, recouvrant au moins toute la surface de la couche de polymères filmogène a), où
   e) la couche protectrice résiste aux matériaux des autres constituants du lamifié et aux matériaux utilisés pour la préparation du lamifié, et
   f) l'adhérence de la couche protectrice d) à la couche de polymères a) est plus faible, du fait de la présence d'un apprêt contradhésif sur le côté revêtu que l'adhérence entre la couche de colle de contact b) et la feuille de support c), caractérisé en ce que
   g) l'apprêt contradhésif s'obtient par un revêtement, qui contient, à titre de constituant essentiel, un sel complexe d'un acide gras comportant de 12 à 22 atomes de carbone, qui est saturé ou qui contient jusqu'à 3 liaisons oléfiniques et qui contient un métal au moins bivalent.

2. Lamifié autocollant suivant la revendication 1, caractérisé en ce que le métal au moins bivalent est un métal de transition.

3. Lamifié autocollant suivant la revendication 2, caractérisé en ce que le métal lourd est le chrome.

4. Lamifié autocollant suivant la revendication 1, caractérisé en ce que la couche protectrice d) est structurée.

5. Lamifié autocollant suivant la revendication 1, caractérisé en ce que la couche protectrice d) se compose d'une feuille, de préférence, en une matière plastique, un papier ou en un article plat textile.

6. Lamifié autocollant suivant une ou plusieurs des revendications 1 à 3, caractérisé en ce que l'adhérence de la couche protectrice d) à la couche de polymères a) est plus faible que l'adhérence entre la couche de colle b) et la feuille de support c).

**7.** Lamifié autocollant suivant une ou plusieurs des revendications 1 à 6, caractérisé en ce que la couche protectrice d) est transparente.

**8.** Lamifié autocollant suivant une ou plusieurs des revendications 1 à 7, caractérisé en ce que la couche de colle b) et/ou la couche protectrice d) sont chacune multicouche.

**9.** Lamifié autocollant suivant une ou plusieurs des revendications 1 à 8, caractérisé en ce que la feuille de support c) est pourvue d'une aide ou d'un auxiliaire d'arrachement.

**10.** Lamifié autocollant suivant une ou plusieurs des revendications 1 à 9, caractérisé en ce que la colle de contact possède un grammage de 20 à 100 $g/m^2$, de préférence, 25 à 75 $g/m^2$.

**11.** Lamifié autocollant suivant une ou plusieurs des revendications 1 à 10, caractérisé en ce que la couche de polymères a) présente une épaisseur de 25 à 200 $\mu m$, de préférence, 60 à 150 $\mu m$.

**12.** Lamifié autocollant suivant une ou plusieurs des revendications 1 à 11, caractérisé en ce que la concentration des plastifiants dans la couche de polymères a) varie de 15 à 25% en poids, de préférence, 17 à 22% en poids, par rapport aux polymères.

**13.** Lamifié autocollant suivant une ou plusieurs des revendications 1 à 12, caractérisé en ce qu'il contient un plastifiant dont la migration ou l'exsudation est extraordinairement faible.

**14.** Lamifié autocollant suivant une ou plusieurs des revendications 1 à 12, caractérisé en ce qu'il contient un ester de l'acide citrique à titre de plastifiant.

**15.** Lamifié autocollant suivant une ou plusieurs des revendications 1 à 13, caractérisé en ce que la couche de polymères a) est multicouche.

**16.** Lamifié autocollant suivant une ou plusieurs des revendications 1 à 15, caractérisé en ce que les couches sont de compositions différentes.

**17.** Lamifié autocollant suivant une ou plusieurs des revendications 1 à 16, caractérisé en ce que la couche de polymères a) se compose au moins en partie de nitrocellulose et/ou d'acrylate, où il s'agit, dans le cas des acrylates, de polymères d'esters de l'acide acrylique ou de l'acide méthacrylique, plus particulièrement, d'alcools comportant de 1 à 4 atomes de carbone, de préférence, 1 ou 2 atomes de carbone et/ou de méthacrylate de diméthylaminoéthyle.

**18.** Lamifié autocollant suivant une ou plusieurs des revendications 1 à 17, caractérisé en ce que la couche de polymères a) est constituée d'un vernis à ongles usuel du commerce qui est dépourvu de camphre.

**19.** Lamifié autocollant suivant une ou plusieurs des revendications 1 à 18, caractérisé en ce que la couche de polymères a) et/ou la couche de colle b) contiennent un composant olfactif.

**20.** Lamifié autocollant suivant une ou plusieurs des revendications 1 à 19, caractérisé en ce que la couche de polymères a) et/ou la couche de colle b) contiennent un principe actif.

**21.** Lamifié autocollant suivant une ou plusieurs des revendications 1 à 20, caractérisé en ce que la couche de polymères a) et/ou la couche de colle b) contiennent des substances à activité antimycotique.

**22.** Lamifié autocollant suivant la revendication 21, caractérisé en ce que la couche de polymères a) et/ou la couche de colle b) contiennent, à titre de principes actifs antimycotiques, du chlotrimazole, du miconazole, du cétoconazole, de l'éconazole, de la naftidine, du fenticlor, de la sulbentine, du tolnaftate et/ou de l'haloprogine ou, à titre de composé antimycotique, une 1-hydroxy-2-pyridone de la formule :

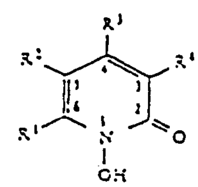

dans laquelle $R^1$ représente un radical hydrocarboné, qui contient de 6 à 9 atomes de carbone, mais cependant dépourvu de liaisons oléfiniques et acétyléniques, l'un des symboles $R^2$ et $R^4$ représente un atome d'hydrogène, cependant que l'autre représente un atome d'hydrogène, le radical méthyle ou éthyle et $R^3$ représente un radical alkyle comportant 1 ou 2 atomes de carbone.

**23.** Lamifié autocollant suivant une ou plusieurs des revendications 1 à 22, caractérisé en ce que la couche de colle b) contient une substance favorisant la pénétration du principe actif dans l'ongle, comme le sulfoxyde de diméthyle ou le diméthylformamide.

**24.** Lamifié autocollant suivant une ou plusieurs des revendications 1 à 23, caractérisé en ce que la couche protectrice d) a été préalablement rendue contradhésive du côté revêtu.

**25.** Lamifié autocollant suivant une ou plusieurs des revendications 1 à 24, caractérisé en ce que la couche de polymères contient un agent tinctorial, un colorant, un pigment ou une combinaison de ceux-ci.

**26.** Utilisation du lamifié suivant une ou plusieurs des revendications 1 à 25 pour l'application à titre d'ongles artificiels sur les ongles des pieds et des doigts.

**Revendications pour les Etats contractants suivants : IE, MC, PT**

**1.** Lamifié autocollant, adaptable ou conformable aux ongles des pieds et des doigts, qui contient :

a) une couche de polymères filmogène,
b) une couche autocollante ou de colle de contact sur cette couche de polymères, et
c) une feuille de support amovible, recouvrant la couche de colle de contact ou autocollante, ainsi que
d) une couche protectrice, recouvrant au moins totalement en surface la couche de polymères filmogène a),
qui se caractérise par les particularités suivantes :
e) la couche de polymères filmogène contient 1 à 30% en poids d'un plastifiant, par rapport aux polymères,
f) la couche protectrice résiste aux autres constituants du lamifié et aux matériaux utilisés pour la préparation du lamifié,
g) l'adhérence de la couche protectrice d) à la couche de polymères a) est plus faible que l'adhérence entre la couche de colle b) et la feuille de support c).

**2.** Lamifié autocollant suivant la revendication 1, caractérisé en ce que la couche protectrice d) est structurée.

**3.** Lamifié autocollant suivant la revendication 1 ou 2, caractérisé en ce que la couche protectrice d) se compose d'une feuille, de préférence, en une matière plastique, un papier ou en un article plat textile.

**4.** Lamifié autocollant suivant une ou plusieurs des revendications 1 à 3, caractérisé en ce que la couche protectrice d) est transparente.

**5.** Lamifié autocollant suivant une ou plusieurs des revendications 1 à 4, caractérisé en ce que la couche de colle b) et/ou la couche protectrice d) sont chacune multicouche.

**6.** Lamifié autocollant suivant une ou plusieurs des revendications 1 à 5, caractérisé en ce que la feuille de support c) est pourvue d'une aide ou d'un auxiliaire d'arrachement.

**7.** Lamifié autocollant suivant une ou plusieurs des revendications 1 à 6, caractérisé en ce que la colle de contact possède un grammage de 20 à 100 $g/m^2$, de préférence, 25 à 75 $g/m^2$.

**8.** Lamifié autocollant suivant une ou plusieurs des revendications 1 à 7, caractérisé en ce que la couche de polymères a) présente une épaisseur de 25 à 200 $\mu m$, de préférence, 60 à 150 $\mu m$.

**9.** Lamifié autocollant suivant une ou plusieurs des revendications 1 à 8, caractérisé en ce que la concentration des plastifiants dans la couche de polymères a) varie de 15 à 25% en poids, de préférence, 17 à 22% en poids, par rapport aux polymères.

**10.** Lamifié autocollant suivant une ou plusieurs des revendications 1 à 9, caractérisé en ce qu'il contient un plastifiant dont la migration ou l'exsudation est extraordinairement faible.

**11.** Lamifié autocollant suivant une ou plusieurs des revendications 1 à 10, caractérisé en ce qu'il contient un ester de l'acide citrique à titre de plastifiant.

**12.** Lamifié autocollant suivant une ou plusieurs des revendications 1 à 11, caractérisé en ce que la couche de polymères a) est multicouche.

**13.** Lamifié autocollant suivant une ou plusieurs des revendications 6 à 12, caractérisé en ce que les couches sont de compositions différentes.

**14.** Lamifié autocollant suivant une ou plusieurs des revendications 1 à 13, caractérisé en ce que la couche de polymères a) se composé au moins en partie de nitrocellulose et/ou d'acrylate, où il s'agit, dans le cas des acrylates, de polymères d'esters de l'acide acrylique ou de l'acide méthacrylique, plus particulièrement, d'alcools comportant de 1 à 4 atomes de carbone, de préférence, 1 ou 2 atomes de carbone et/ou de méthacrylate de diméthylaminoéthyle.

**15.** Lamifié autocollant suivant une ou plusieurs des revendications 1 à 14, caractérisé en ce que la couche de polymères a) est constituée d'un vernis à ongles usuel du commerce qui est dépourvu de camphre.

**16.** Lamifié autocollant suivant une ou plusieurs des revendications 1 à 15, caractérisé en ce que la couche de polymères a) et/ou la couche de colle b) contiennent un composant olfactif.

**17.** Lamifié autocollant suivant une ou plusieurs des revendications 1 à 16, caractérisé en ce que la couche de polymères a) et/ou la couche de colle b) contiennent un principe actif.

**18.** Lamifié autocollant suivant une ou plusieurs des revendications 1 à 17, caractérisé en ce que la couche de polymères a) et/ou la couche de colle b) contiennent des substances à activité antimycotique.

**19.** Lamifié autocollant suivant la revendication 18, caractérisé en ce que la couche de polymères a) et/ou la couche de colle b) contiennent, à titre de principes actifs antimycotiques, du chlotrimazole, du miconazole, du cétoconazole, de l'éconazole, de la naftidine, du fenticlor, de la sulbentine, du tolnaftate et/ou de l'haloprogine ou, à titre de composé antimycotique, une 1-hydroxy-2-pyridone de la formule :

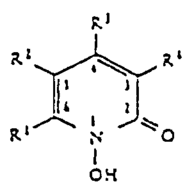

dans laquelle $R^1$ représente un radical hydrocarboné, qui contient de 6 à 9 atomes de carbone, mais cependant dépourvu de liaisons oléfiniques et acétyléniques, l'un des symboles $R^2$ et $R^4$ représente un atome d'hydrogène, cependant que l'autre représente un atome d'hydrogène, le radical méthyle ou éthyle et $R^3$ représente un radical alkyle comportant 1 ou 2 atomes de carbone.

**20.** Lamifié autocollant suivant une ou plusieurs des revendications 1 à 19, caractérisé en ce que la couche de colle b) contient une substance favorisant la pénétration du principe actif dans l'ongle, comme le sulfoxyde de diméthyle ou le diméthylformamide.

**21.** Lamifié autocollant suivant une ou plusieurs des revendications 1 à 20, caractérisé en ce que la couche protectrice d) a été préalablement rendue contradhésive du côté revêtu.

**22.** Lamifié autocollant suivant la revendication 21, caractérisé en ce que l'apprêt contradhésif est obtenu par l'intermédiaire d'un revêtement qui contient, à titre de constituant essentiel, un sel complexe d'un acide gras possédant de 12 à 22 atomes de carbone, qui est saturé, ou qui contient jusqu'à 3 liaisons oléfiniques et d'un métal au moins bivalent.

**23.** Lamifié autocollant suivant la revendication 22, caractérisé en ce que le métal au moins bivalent est un métal de transition lourd.

**24.** Lamifié autocollant suivant la revendication 23, caractérisé en ce que le métal lourd est le chrome.

**25.** Lamifié autocollant suivant la revendication 21, caractérisé en ce que l'apprêt contradhésif est obtenu par le revêtement à l'aide d'une silicone convenant à cette fin.

**26.** Lamifié autocollant suivant une ou plusieurs des revendications 1 à 25, caractérisé en ce que l'agent tinctorial est un colorant ou un pigment, ou une combinaison de ceux-ci.

**27.** Procédé de préparation d'un lamifié autocollant, adaptable ou conformable aux ongles des pieds ou des doigts, suivant une ou plusieurs des revendications 1 à 26, caractérisé en ce que l'on applique un vernis qui contient au moins un plastifiant et au moins un polymère filmogène, dissous ou dispersés dans un solvant organique et avantageusement aussi un agent tinctorial, sur une couche protectrice amovible d), qui possède la même taille que le lamifié, on sèche le vernis sous récupération du solvant et on lamifie la couche protectrice d) revêtue du vernis, côté vernis, avec un film de colle et une feuille de support c).

**28.** Utilisation du lamifié suivant une ou plusieurs des revendications 1 à 26 et du lamifié obtenu par mise en oeuvre du procédé suivant la revendication 27 pour l'application à titre d'ongles artificiels sur les ongles des pieds et des doigts.

FIG. 1

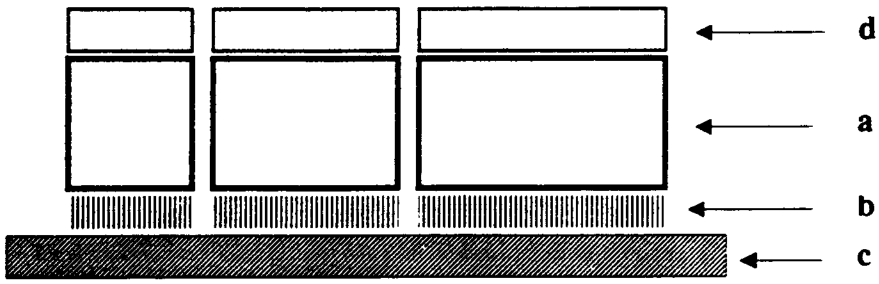


FIG. 2

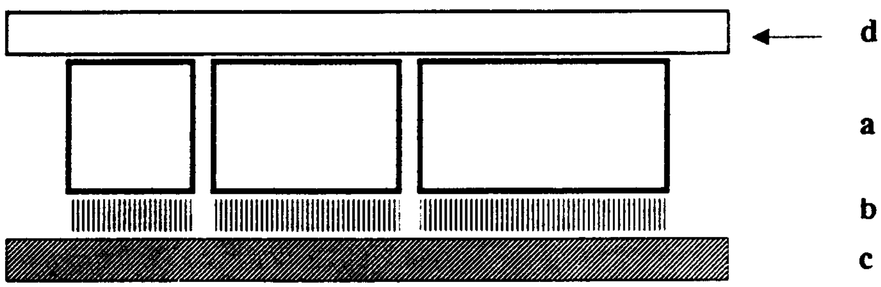


FIG. 3